# EUROPÄISCHE PATENTSCHRIFT

(11) **EP 1 411 906 B1**
(45) Veröffentlichungstag und Bekanntmachung des Hinweises auf die Patenterteilung: **19.10.2005**
(21) Anmeldenummer: 02776521.3
(22) Anmeldetag: 06.06.2002
(51) Int. Cl.: A61K 9/70

(54) **TTS MIT ABZIEHFOLIE MIT 1-DIMENSIONAL HERABGESETZTER REISSFESTIGKEIT**
TTS WITH PEEL-OFF FILM WITH REDUCED RESISTANCE TO TEARING
SYSTEME THERAPEUTIQUE TRANSDERMIQUE COMPORTANT UNE PELLICULE DETACHABLE DONT LA RESISTANCE AU DECHIREMENT EST REDUITE

(30) Priorität: 07.06.2001 DE 10127455
(43) Veröffentlichungstag der Anmeldung: 28.04.2004
(73) Patentinhaber: HEXAL AG, 83607 Holzkirchen (DE)
(72) Erfinder: KIBELE, Ralf, 83607 Holzkirchen (DE)
(74) Vertreter: Boeters, Hans Dietrich
(86) Internationale Anmeldenummer: PCT/EP2002/006215
(87) Internationale Veröffentlichungsnummer: WO 2002/098395

(56) Entgegenhaltungen:
- WO-A-01/47502
- WO-A-98/25257

## Beschreibung

Abziehhilfen bzw. Applizierhilfen sind bei auf dem Markt befindlichen transdermalen therapeutischen Systemen weit verbreitet. Beispielhaft sei auf PCT/EP97/06 799 verwiesen. Abziehhilfen sollen dem Anwender ermöglichen, das transdermale therapeutische System leichter zu applizieren. Abziehhilfen sind daher zumindest sinnvolles Element des Systems, da durch Abziehhilfen die Anwendung des Systems erleichtert wird.

Bei empfindlichen transdermalen therapeutischen Systemen kann allerdings durch die direkte Ausbildung der Abziehhilfe a) das System beschädigt oder b) die Stabilität des Systems negativ beeinflußt werden.

Fall a) tritt insbesondere bei transdermalen therapeutischen Systemen vom Reservoir-Typ ein, bei denen durch eine Verletzung der Membran die wirkstoffhaltige Lösung aus dem Reservoir austreten kann, wodurch das System unbrauchbar wird. Fall b) tritt insbesondere bei transdermalen therapeutischen Systemen vom Matrix-Typ auf, bei denen systembedingt das System meistens mit dem jeweiligen Wirkstoff übersättigt ist. So konnte beobachtet werden, daß es längs des Schnitts bzw. der Stanzung der Abziehhilfe verstärkt zur Kristallisation des Wirkstoffs kommt.

Wird bei transdermalen therapeutischen Systemen vom Reservoir-Typ keine Abziehhilfe vorgesehen, so wird die Anwendung erheblich erschwert.

Für Fall b) wird häufig zunächst ein Laminat mit einer ersten Abziehfolie hergestellt, die im Verlauf der Konfektionierung abgezogen und durch eine zweite Abziehfolie mit vorgestanzter Abziehhilfe ersetzt wird. Die Herstellung eines transdermalen therapeutischen Systems vom Matrix-Typ mit erster und zweiter Abziehfolie führt jedoch zu erhöhten Kosten, einem erhöhten maschinellen Aufwand und erhöhten Arbeitszeiten.

Zur Verbesserung des Stands der Technik wird nun erfindungsgemäß ein transdermales therapeutisches System (TTS) mit einer Abziehfolie vorgesehen, die in einer Richtung (1-dimensional) eine herabgesetzte Reißfestigkeit bzw. Weiterreißfesteigkeit aufweist.

Geeignete Abziehfolien haben beispielsweise eine Weiterreißfestigkeit (1-dimensional) von 10 bis 50 und insbesondere 15 bis 25 Newton/mm.

Erfindungsgemäß kann die Abziehfolie allseitig über die Peripherie des transdermalen therapeutischen Systems bzw. der restlichen Bauelemente des transdermalen therapeutischen Systems überstehen. Unter restlichen Bauelementen werden im vorliegenden Zusammenhang alle Bauelemente eines transdermalen therapeutischen Systems verstanden, abgesehen von der Abziehfolie.

Erfindungsgemäß kann der allseitige Überstand der Abziehfolie eine Abziehhilfe zum Einreißen der Abziehfolie in Richtung der herabgesetzten Reißfestigkeit aufweisen.

Erfindungsgemäß kann es sich bei der Abziehhilfe um einen Einschnitt, eine Einkerbung, eine Perforation oder eine Schwachstelle handeln. Diese Abziehhilfe kann durch Stanzen vorgesehen worden sein.

Erfindungsgemäß kann die Abziehhilfe vom Rand der Abziehfolie ausgehend vorgesehen sein.

Erfindungsgemäß kann die Abziehhilfe bis vor das transdermale therapeutische System (seine restlichen Bauelemente) geführt sein.

Erfindungsgemäß kann die Abziehfolie zusätzlich zu einer ersten Abziehhilfe eine weitere Abziehhilfe aufweisen, die auf der gegenüberliegenden Seite des transdermalen therapeutischen Systems (seiner restlichen Bauelemente) vorgesehen ist.

Erfindungsgemäß können die beiden Abziehhilfen derart zueinander angeordnet sein, daß das Ende eines Risses, der von einer der beiden Abziehhilfen ausgeht, die andere Abziehhilfe beim Abziehen der Abziehfolie durchtrennt.

Erfindungsgemäß kann es sich bei der Abziehfolie um eine biaxial orientierte oder gereckte Folie oder um einen Folienverbund mit einer biaxial orientierten oder gereckten Folie handeln.

Erfindungsgemäß kann die einlagige Abziehfolie oder mindestens eine Folie des Folienverbundes eine Polypropylen- oder Polyethylen-Folie sein (PP- oder PE-Folie), insbesondere eine geblasene Polypropylen-Folie. Geblasene Polypropylen-Folien (PPb-Folien) sind gegossenen PP-Folien (PPc-Folien) oder Polyethylen-Folien als Grundmaterial überlegen, da die geblasenen Polypropylen-Folien eine höhere Temperaturstabilität (geringerer Schrumpf) und in der Regel eine gleichmäßigere Schichtdicke aufweisen.

Biaxial gereckte Polypropylen-Folien besitzen eine hohe Querreiß-Festigkeit, aber praktisch keine Längsweiter-Reißfestigkeit. Diese Eigenschaft läßt sich erfindungsgemäß vorteilhaft für Abziehhilfen ausnutzen.

Erfindungsgemäß kann es sich bei dem Folienverbund um einen PP/PE-, PP/PET-, PP/PS-, PP/EVA-, PP/EVOH-, PP/Papier-, PP/PVC-, PP/PVDC- oder PP/OPA-Verbund handeln.

Für einen PP/PET-Verbund ist eine PET-Stärke von maximal etwa 15 µm vorzuziehen.

Bei einem Folienverbund ist die Hauptkomponente von der Stärke her bevorzugt PP. Erfindungsgemäß ist die Polypropylen-Komponente des Folienverbundes genauso dick oder dicker als die anderen Komponenten des Folienverbundes.

Erfindungsgemäß kann ein transdermales therapeutisches System mit einer Abziehfolie einer Dicke von 15 bis 300 *µ*m und insbesondere von 50 bis 150 *µ*m vorgesehen werden.

Erfindungsgemäß kann ein transdermales therapeutisches System vorgesehen werden, bei dem die Abziehfolie als einlagige Abziehfolie oder als Folienverbund mit einer Trennbeschichtung versehen ist. Bei der Trennbeschichtung kann es sich um eine Silikonisierung oder um eine Fluorosilikonisierung handeln.

Erfindungsgemäß wird es also durch einen einfachen geraden Schnitt, der an einer beliebigen Position am Rande des Überstandes der Abziehfolie längs zur Laufrichtung bzw. in Richtung herabgesetzter Weiterreißfestigkeit vorgesehen wird, in vorteilhafter Weise möglich, daß sich eine Abziehhilfe vorsehen läßt, ohne daß das transdermale therapeutische System verletzt wird. Durch einen einfachen Zug kann die Abziehfolie definiert in zwei Teile bei Applikation getrennt werden.

Nachstehend wird die Erfindung anhand von Figuren und Beispielen näher erläutert.

Es zeigen:
Figur 1 ein TTS vom Reservoir-Typ des Stands der Technik;
Figur 2 ein TTS vom Matrix-Typ des Stands der Technik;
Figur 3 verschiedene Stadien bei der Herstellung von erfindungsgemäßen transdermalen therapeutischen Systemen;
Figur 4 ein erfindungsgemäßes TTS mit einer Abziehhilfe;
Figur 5 ein erfindungsgemäßes TTS mit einer Abziehhilfe;
Figur 6 ein erfindungsgemäßes TTS mit zwei Abziehhilfen;
Figur 7 ein erfindungsgemäßes TTS mit zwei Abziehhilfen; und
Figur 8 ein Stadium bei der Herstellung von erfindungsgemäßen therapeutischen Systemen mit jeweils einer Abziehhilfe.

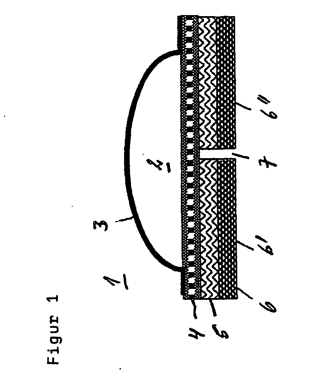

Bei dem in Figur 1 dargestellten transdermalen therapeutischen System 1 wird das Reservoir 2, das den zu applizierenden Wirkstoff enthält, von einer Abdeckfolie 3 und einer Membran 4 eingeschlossen. Auf die flächige Membran 4 ist eine selbstklebende Schicht 5 aufgebracht, die bis zur Applikation des TTS 1 mit einer Abziehfolie 6 geschützt ist. Die Abziehfolie 6 weist einen quer über die Abziehfolie 6 laufenden Schnitt 7 auf, der bis in die selbstklebende Schicht 5 hineingeführt sein kann. Dieser Schnitt 7 kann beispielsweise gemäß PCT/EP97/06799 ausgebildet sein. Der Schnitt 7 gestattet es, das TTS 1 bei Applikation derart leicht durchzubiegen, daß die Abziehfolie 6 leicht konvex vorsteht und die Schnittkanten des Schnitts 7 derart vorragen, daß sie ergriffen und die beiden Teile der Abziehfolie 6', 6" von der Klebschicht 5 abgezogen werden können, so daß die Klebschicht 5 freikommt und das TTS 1 appliziert werden kann.

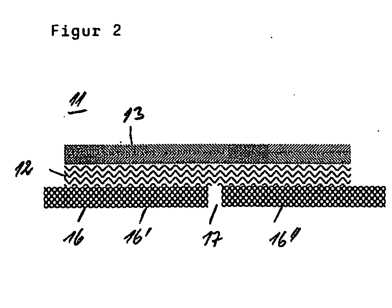

Bei dem in Figur 2 dargestellten TTS 11 vom Matrix-Typ des Stands der Technik ist eine wirkstoffhaltige Matrixschicht 12 auf eine Abdeckfolie 13 aufgebracht und auf ihrer der Abdeckfolie 13 abgewandten Seite mit einer Abziehfolie 16 abgedeckt. Die Abziehfolie 16 ist wiederum mit einem durchgehenden Einschnitt 17 versehen, der es ermöglicht, die Abziehfolie 16 hälftig abzuziehen. Die beiden Hälften 16', 16" der Abziehfolie 16 entsprechen den Hälften 6', 6" der Abziehfolie 6 des in Figur 1 dargestellten TTS 1.

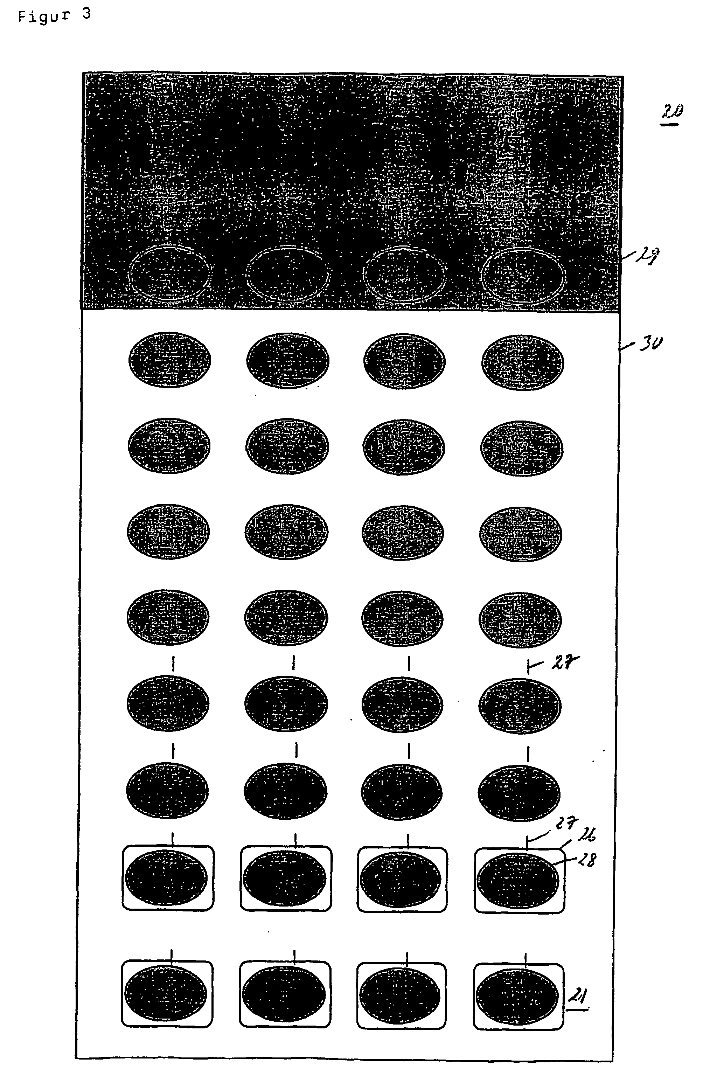

Figur 3 soll die Herstellung eines erfindungsgemäßen TTS vom Matrix-Typ erläutern. Die Bahn 20 kann als dreilagiges Endlosband vorgesehen sein (Bereich 29), wobei die oberste Lage der Bahn 2o für die Abdeckfolien, die mittlere Lage der Bahn 20 für die Matrixschichten und die unterste Lage der Bahn 20 für die Abziehfolien der herzustellenden transdermalen Systeme vorgesehen ist. Die dreilagige Bahn 20 wird mit (in vier Zeilen angeordneten) etwa ellipsenförmigen Einschnitten versehen, die durch die obere und mittlere Lage der Bahn 20 geführt sind und einzelne Pflaster 28 definieren. Danach werden die obere und die mittlere Lage der Bahn 20 derart abgezogen, daß nur noch die untere Lage 30 mit den vorgebildeten (und in vier Zeilen angeordneten) Pflastern 28 verbleibt. In einem weiteren Schritt wird die untere Lage 30 der Bahn 20 mit Einschnitten 27 versehen, und zwar jeweils einem Einschnitt 27 pro herzustellendes Pflaster 28. In einem weiteren Schritt werden aus der unteren Bahn 30 etwa rechteckige Abziehfolien 26 derart ausgestanzt, daß die einzelnen Abziehfolien 26 allseitig über die Peripherie der aus Abdeckfolie und Matrix gebildeten Pflaster 28 (Pflaster ohne Abziehfolie 26) überstehen. Aus Figur 3 kann man entnehmen, daß die Einschnitte 27 derart vorgesehen sind, daß sie jeweils in Richtung auf das ihnen zugeordnete Pflaster 28 durch den Rand der Abziehfolie 26 hindurchgeführt sind, jedoch die Peripherie des ihnen jeweils zugeordneten Pflasters 28 nicht erreichen.

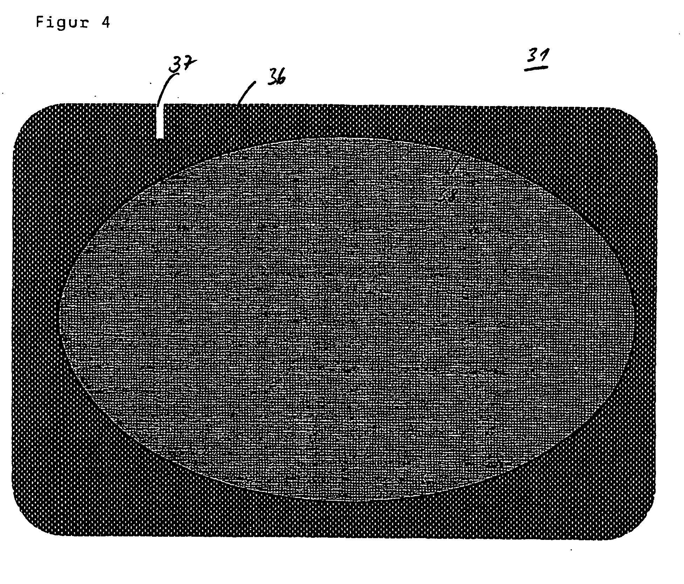

Dieser Sachverhalt ist detailliert Figur 4 zu entnehmen. In Figur 4 ist ein in Draufsicht etwa ellipsenförmiges transdermales System 31 vom Matrix-Typ dargestellt, das aus einer Abdeckfolie und einer darunterliegenden selbstklebenden Matrixschicht gebildet wird, wobei diese Matrixschicht von einer allseitig überstehenden Abziehfolie 36 abgedeckt ist. Diese Abziehfolie 36 ist mit einem Einschnitt 37 versehen, der vom Rand der Abziehfolie 36 in die Abziehfolie 36 einschneidet, jedoch das Pflaster 38 (Pflaster ohne Abziehfolie) nicht erreicht. Erfindungsgemäß weist die Abziehfolie 36 in einer Richtung (1-dimensional) eine herabgesetzte Reißfestigkeit auf. Da der Einschnitt 37 in Richtung der herabgesetzten Reißfestigkeit geführt ist, kann die Abziehfolie 36 bei Applikation des transdermalen Systems 31 mit Hilfe des Einschnitts 37 unter dem Pflaster 38 hindurch derart durchtrennt werden, daß sich die beiden Teile der Abziehfolie 36, die beim Einreißen des Einschnitts 37 zu beiden Seiten des Risses zurückbleiben, leicht vom Pflaster 38 abheben lassen.

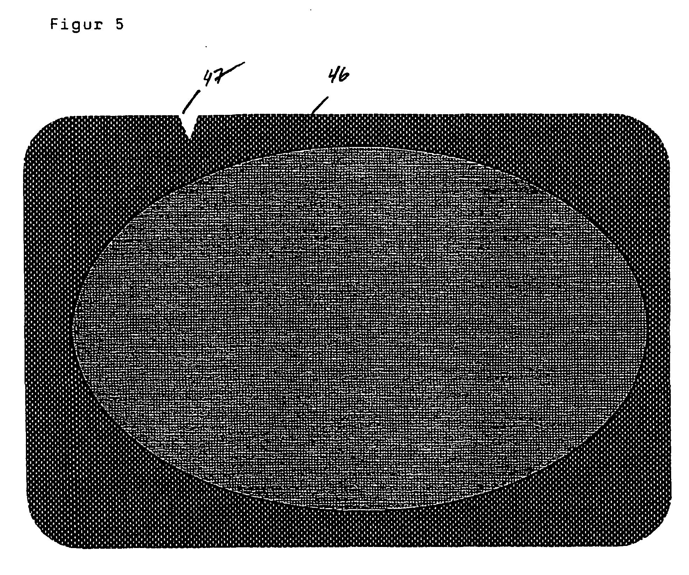

Figur 5 zeigt ein erfindungsgemäßes TTS, bei dem die Abziehhilfe 47 als keilförmiger Einschnitt in die Abziehfolie 46 ausgebildet ist.

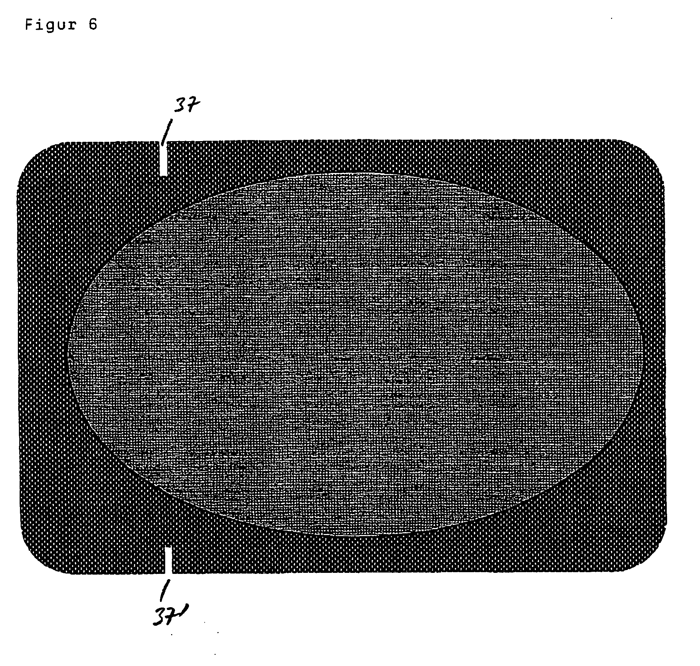

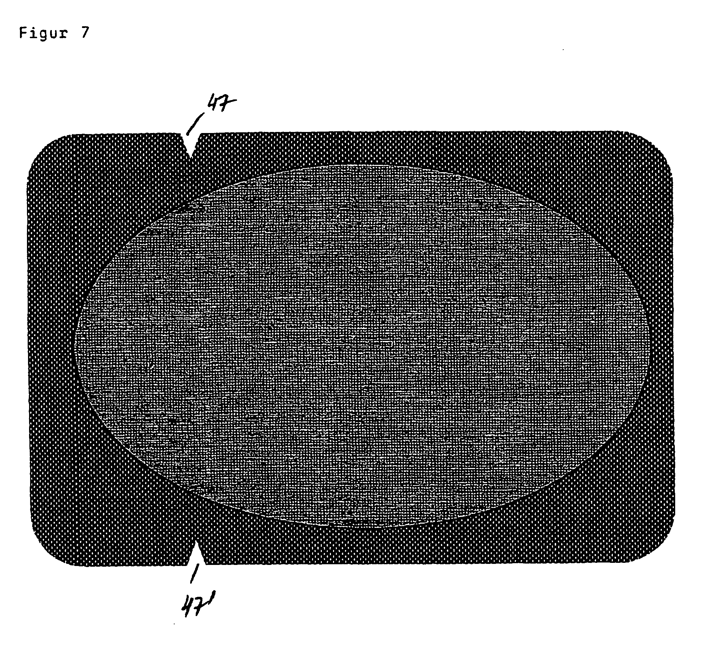

Die Figuren 6 und 7 zeigen transdermale therapeutische Systeme mit jeweils zwei Abziehhilfen (37, 37' in Figur 6 und 47, 47' in Figur 7), die jeweils am Rand der Abziehfolie vorgesehen sind, so daß sie jeweils das aus Abdeckfolie und Matrix gebildete Pflaster nicht erreichen. Die Abziehhilfen 37, 37' und 47, 47' sind also paarig und derart angeordnet, daß ein Riß, der von einer der Abziehhilfen ausgeht und in Richtung der herabgesetzten Reißfestigkeit der Abziehfolie geführt ist, jeweils die gegenüberliegende Abziehhilfe durchtrennt.

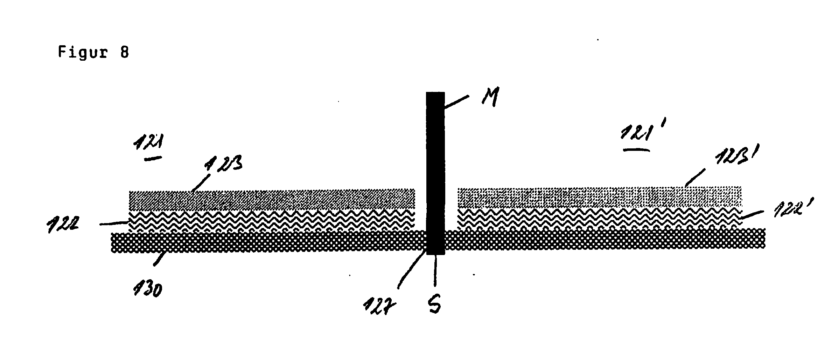

Figur 8 zeigt zwei transdermale therapeutische Systeme 121, 121', die jeweils aus einer Abdeckfolie 123, 123' und einer selbstklebenden Matrixschicht 122, 122' zweilagig gebildet sind. Die beiden transdermalen Systeme 121, 121' sind auf einer gemeinsamen Bahn 130 vorgesehen, aus der gemäß Figur 3 Abziehfolien herauszustanzen sind, die die Pflaster 123, 123' tragen und allseitig überragen. Figur 8 zeigt einen Schritt bei der TTS-Herstellung, der dem Herausstanzen der Abziehfolien gemäß Figur 3 vorangeht, und zwar wird mit einem Meißel M, der eine in der Zeichnungsebene liegende Schnittkante S aufweist, ein Einschnitt 127 angebracht, der lediglich durch die Bahn 130, jedoch nicht durch die Pflaster 123, 123' geführt ist und diese Pflaster 123, 123' unversehrt läßt. Im dargestellten Ausführungsbeispiel werden mit einem einzigen Einschnitt 127 zugleich jeweils eine Abziehhilfe für das Pflaster 123 und das Pflaster 123' vorgesehen, bevor die Abziehfolien für die beiden Pflaster 123, 123' aus der noch gemeinsamen Bahn 130 ausgestanzt werden.

## Patentansprüche

1. Transdermales therapeutisches System (TTS) mit einer Abziehfolie, die in einer Richtung (1-dimensional) eine herabgesetzte Reißfestigkeit aufweist.

2. Transdermales therapeutisches System nach Anspruch 1, **dadurch gekennzeichnet, daß** die Abziehfolie allseitig über die Peripherie des transdermalen therapeutischen Systems (der restlichen Bauelemente des transdermalen therapeutischen Systems) übersteht.

3. Transdermales therapeutisches System nach Anspruch 1 oder 2, **dadurch gekennzeichnet, daß** der allseitige Überstand der Abziehfolie eine Abziehhilfe zum Einreißen der Abziehfolie in Richtung der herabgesetzten Reißfestigkeit aufweist.

4. Transdermales therapeutisches System nach einem der vorhergehenden Ansprüche, **dadurch gekennzeichnet, daß** es sich bei der Abziehhilfe um einen Einschnitt, eine Einkerbung, eine Perforation oder eine Schwachstelle handelt.

5. Transdermales therapeutisches System nach einem der vorhergehenden Ansprüche, **dadurch gekennzeichnet, daß** die Abziehhilfe vom Rand der Abziehfolie ausgehend vorgesehen ist.

6. Transdermales therapeutisches System nach einem der vorhergehenden Ansprüche, **dadurch gekennzeichnet, daß** die Abziehhilfe bis vor das transdermale therapeutische System (die restlichen Bauelemente des transdermalen therapeutischen Systems) geführt ist.

7. Transdermales therapeutisches System nach einem der vorhergehenden Ansprüche, **dadurch gekennzeichnet, daß** der allseitige Überstand der Abziehfolie zusätzlich zu einer ersten Abziehhilfe eine weitere Abziehhilfe aufweist, die auf der gegenüberliegenden Seite des transdermalen therapeutischen Systems (der restlichen Bauelemente des transdermalen therapeutischen Systems) vorgesehen ist.

8. Transdermales therapeutisches System nach einem der vorhergehenden Ansprüche, **dadurch gekennzeichnet, daß** erste und zweite Abziehhilfe derart zueinander angeordnet sind, daß das Ende eines Risses, der von einer der beiden Abziehhilfen ausgeht, die andere Abziehhilfe beim Abziehen der Abziehfolie durchtrennt.

9. Transdermales therapeutisches System nach einem der vorhergehenden Ansprüche, **dadurch gekennzeichnet, daß** die Abziehfolie eine biaxial gereckte Folie oder ein Folienverbund mit einer biaxial gereckten Folie ist.

10. Transdermales therapeutisches System nach einem der vorhergehenden Ansprüche, **dadurch gekennzeichnet, daß** die einlagige Abziehfolie oder daß mindestens eine Folie des Folienverbandes eine Polypropylen- oder Polyethylen-Folie ist (PP- oder PE-Folie), insbesondere eine geblasene Polypropylen-Folie.

11. Transdermales therapeutisches System nach einem der vorhergehenden Ansprüche, **dadurch gekennzeichnet, daß** es sich bei dem Folienverbund um einen PP/PE-, PP/PET-, PP/PS-, PP/EVA-, PP/EVOH-, PP/Papier-, PP/PVC-, PP/PVDC- oder PP/OPA-Verbund handelt.

12. Transdermales therapeutisches System nach Anspruch 11, **dadurch gekennzeichnet, daß** die Polypropylen-Komponente (PP-Komponente) des Folienverbundes genauso dick oder dicker als die andere Komponente des Folienverbundes ist.

13. Transdermales therapeutisches System nach einem der vorhergehenden Ansprüche, **gekennzeichnet durch** eine Abziehfolie einer Dicke von 15 bis 300 µm, insbesondere von 50 bis 150 µm.

14. Transdermales therapeutisches System nach einem der vorhergehenden Ansprüche, **dadurch gekennzeichnet, daß** die Abziehfolie als einlagige Abziehfolie oder als Folienverbund mit einer Trennbeschichtung versehen ist.

15. Transdermales therapeutisches System nach Anspruch 14, **dadurch gekennzeichnet, daß** es sich bei der Trennbeschichtung um eine Silikonisierung oder Fluorosilikonisierung handelt.

## Claims

1. A transdermal therapeutic system (TTS) having a peel-off film that has a reduced resistance to tearing in one direction (1-dimensionally).

2. The transdermal therapeutic system according to claim 1, **characterised in that** the peel-off film projects all round beyond the periphery of the transdermal therapeutic system (the remainder of the construction elements of the transdermal therapeutic system).

3. The transdermal therapeutic system according to claim 1 or 2, **characterised in that** the all-round projection of the peel-off film has a peel-off aid for tearing the peel-off film in the direction of the reduced resistance to tearing.

4. The transdermal therapeutic system according to any one of the preceding claims, **characterised in that** the peel-off aid is an incision, a notch, a perforation or a weak site.

5. The transdermal therapeutic system according to any one of the preceding claims, **characterised in that** the peel-off aid is provided starting at the edge of the peel-off film.

6. The transdermal therapeutic system according to any one of the preceding claims, **characterised in that** the peel-off aid extends up to the transdermal therapeutic system (the remainder of the construction elements of the transdermal therapeutic system).

7. The transdermal therapeutic system according to any one of the preceding claims, **characterised in that** the all-round projection of the peel-off film has, in addition to a first peel-off aid, a further peel-off aid which is provided on the opposite side of the transdermal therapeutic system (the remainder of the construction elements of the transdermal therapeutic system).

8. The transdermal therapeutic system according to any one of the preceding claims, **characterised in that** the first and the second peel-off aid are so arranged relative to each other that the end of a tear that begins at one of the two peel-off aids divides the other peel-off aid upon peeling-off of the peel-off film.

9. The transdermal therapeutic system according to any one of the preceding claims, **characterised in that** the peel-off film is a biaxially stretched film or a film composite comprising a biaxially stretched film.

10. The transdermal therapeutic system according to any one of the preceding claims, **characterised in that** the single-layer peel-off film or at least one film of the film composite is a polypropylene or polyethylene film (PP or PE film), especially a blown polypropylene film.

11. The transdermal therapeutic system according to any one of the preceding claims, **characterised in that** the film composite is a PP/PE, PP/PET, PP/PS, PP/EVA, PP/EVOH, PP/paper, PP/PVC, PP/PVDC or PP/OPA composite.

12. The transdermal therapeutic system according to claim 11, **characterised in that** the polypropylene component (PP component) of the film composite is as thick or thicker than the other component of the film composite.

13. The transdermal therapeutic system according to any one of the preceding claims, **characterised by** a peel-off film having a thickness of from 15 to 300 µm, especially from 50 to 150 µm.

14. The transdermal therapeutic system according to any one of the preceding claims, **characterised in that** the peel-off film in the form of a single-layer peel-off film or in the form of a film composite is provided with a separation coating.

15. The transdermal therapeutic system according to claim 14, **characterised in that** the separation coating is a silicone treatment or a fluorosilicone treatment.

## Revendications

1. Système thérapeutique transdermique (STT) comportant une pellicule détachable qui présente une résistance au déchirement réduite dans un sens (unidimensionnelle).

2. Système thérapeutique transdermique selon la revendication 1, **caractérisé en ce que** la pellicule détachable dépasse de tous les côtés sur la périphérie du système thérapeutique transdermique (des éléments constitutifs restants du système thérapeutique transdermique).

3. Système thérapeutique transdermique selon la revendication 1 ou 2, **caractérisé en ce que** le dépassement de tous les côtés de la pellicule détachable présente une aide au détachement pour déchirer la pellicule détachable dans le sens de la résistance au déchirement réduite.

4. Système thérapeutique transdermique selon l'une des revendications précédentes, **caractérisé en ce qu'**il s'agit, pour l'aide au détachement, d'une encoche, d'une rainure, d'une perforation ou d'un point faible.

5. Système thérapeutique transdermique selon l'une des revendications précédentes, **caractérisé en ce que** l'aide au détachement est prévue à partir du bord de la pellicule détachable.

6. Système thérapeutique transdermique selon l'une des revendications précédentes, **caractérisé en ce que** l'aide au détachement est amenée jusque devant le système thérapeutique transdermique (les éléments constitutifs restants du système thérapeutique transdermique).

7. Système thérapeutique transdermique selon l'une des revendications précédentes, **caractérisé en ce que** le dépassement de tous les côtés de la pellicule détachable présente, en plus d'une première aide au détachement, une autre aide au détachement, qui est prévue sur le côté du système thérapeutique transdermique (des éléments constitutifs restants du système thérapeutique transdermique) qui se trouve en face.

8. Système thérapeutique transdermique selon l'une des revendications précédentes, **caractérisé en ce que** les première et seconde aides au détachement sont agencées l'une par rapport à l'autre de manière à ce que l'extrémité d'une fente, qui part de l'une des deux aides au détachement, sectionne l'autre aide au détachement lors du détachement de la pellicule détachable.

9. Système thérapeutique transdermique selon l'une des revendications précédentes, **caractérisé en ce que** la pellicule détachable est une pellicule étirée biaxialement ou une combinaison de pellicules avec une pellicule étirée biaxialement.

10. Système thérapeutique transdermique selon l'une des revendications précédentes, **caractérisé en ce que** la pellicule détachable monocouche ou **en ce qu'**au moins une pellicule de l'assemblage de pellicules est une pellicule en polypropylène ou en polyéthylène (pellicule PP ou PE), en particulier une pellicule en polypropylène soufflée.

11. Système thérapeutique transdermique selon l'une des revendications précédentes, **caractérisé en ce qu'**il s'agit, pour la combinaison de pellicules, d'une combinaison PP/PE, PP/PET, PP/PS, PP/EVA, PP/EVOH, PP/papier, PP/PVC, PP/PVDC ou PP/OPA.

12. Système thérapeutique transdermique selon la revendication 11, **caractérisé en ce que** les composants en polypropylène (composants PP) de la combinaison de pellicules sont aussi épais ou plus épais que les autres composants de la combinaison de pellicules.

13. Système thérapeutique transdermique selon l'une des revendications précédentes, **caractérisé par** une pellicule détachable d'une épaisseur de 15 à 300 µm, en particulier de 50 à 150 µm.

14. Système thérapeutique transdermique selon l'une des revendications précédentes, **caractérisé en ce que** la pellicule détachable est prévue sous forme de pellicule détachable monocouche ou de combinaison de pellicules avec un revêtement de séparation.

15. Système thérapeutique transdermique selon la revendication 14, **caractérisé en ce qu'**il s'agit, pour le revêtement de séparation, d'un traitement à la silicone ou d'un traitement à la fluorosilicone.
